Europäisches Patentamt

**(19)** European Patent Office

Office européen des brevets

**(11)** Publication number: **0 199 857**

**A1**

**(12)** EUROPEAN PATENT APPLICATION

**(21)** Application number: 85201735.9

**(22)** Date of filing: 30.11.85

**(51)** Int. Cl.⁴: **A 61 L 15/01**
A 61 L 15/06, A 61 L 15/03
A 61 F 13/00, B 32 B 27/12
C 08 G 18/67

**(30)** Priority: 25.04.85 US 726809

**(43)** Date of publication of application:
05.11.86 Bulletin 86/45

**(84)** Designated Contracting States:
AT BE CH DE FR GB IT LI LU NL SE

**(71)** Applicant: THERMEDICS, INC.
470 Wildwood Street P.O. Box 2999
Woburn Massachusetts(US)

**(72)** Inventor: Szycher, Michael
2, Durham Drive
Lynnfield Massachusetts 01940(US)

**(72)** Inventor: Rolfe, Jonathan L.
82, Bay Road
North Easton Massachusetts 002356(US)

**(74)** Representative: Kupecz, Arpad et al,
Octrooibureau Los en Stigter B.V. Postbox 20052
NL-1000 HB Amsterdam(NL)

**(54)** Anisotropic wound dressing.

**(57)** An anisotropic wound dressing comprised of a knitted reinforcing fabric which is sandwiched between a crosslinked aliphatic polyurethane. The wound dressing is fabricated by dipping the fabric into a polyurethane-forming coating solution to form a thin film which coats the fibers of the fabric and fills the interstices with a film or layer. The film which coats the fabric is cured by ultraviolet radiation and one side of the film is coated with pressure sensitive adhesive to form a wound dressing. In the most preferred embodiments, the knitted reinforcing fabric is an anisotropic fabric formed with a basic stitch construction to create equally spaced and sized hexagonal interstices. Because of the fabric reinforcement, the resulting oxygen permeable product can be made thin, and yet be anisotropic and strong. The resulting product does not wrinkle easily and holds its shape so it is also easily applied.

EP 0 199 857 A1

ANISOTROPIC WOUND DRESSING

CROSS-REFERENCE TO A RELATED APPLICATION

This application is a continuation-in-part of prior application Serial No. 670,810, filed November 13, 1984, entitled "Drug Dispensing Wound Dressing."

BACKGROUND OF THE INVENTION

There has long been a need for a wound dressing which is thin, soft, pliable, elastic, oxygen permeable, yet high in tensile strength and abrasion resistance and which does not promote the growth of bacteria.

Presently available bandages made of materials such as cotton are undesirable because they retain water, serve as growth mediums for bacteria, and soak up tissue pieces and blood which clots, causing adhesion to the wound and trauma during removal.

Bandages made of plastic materials to decrease the undesirable water absorption of cotton wound dressings are available. Unfortunately, problems due to the lack of oxygen transmission through the plastic result from the use of many plastic materials. Indeed, holes are punched through the plastic covering to allow the transmission of some oxygen to the skin below. Such constructions do not provide a barrier to low surface tension aqueous solutions, e.g. washing-up liquid (which will also allow bacteria to penetrate). Silicone coatings have been applied to the area of the bandage adjacent to the wound to prevent adhesion. These coatings do not significantly decrease the problem of

0199857

the bandage sticking to the wound, and do nothing to reduce the blocked oxygen problem.

In further attempts to overcome the adhesion and permeability problems, polyurethane and other plastic dressings were tried. For example U.S. Patent No. 3,975,567 to Lock discloses a pressure and heat-treated polyurethane foam which is lyophilic and U.S. Patent No. 3,870,593 to Elton et al. discloses a polymeric film comprised of finely divided particles of non-hygroscopic inorganic salt dispersed in a suitable polymer.

Other polyurethanes which polymerize upon exposure to ultraviolet light were also developed. The majority of these UV-curable polyurethanes were designed for use as orthopedic casts, for example, U.S. Patent No. 4,209,605 discloses such a cast. None of these compositions managed to combine the properties of softness, oxygen and water vapor permeability, flexibility, and thixotropy.

The desired material for use as a wound dressing or bandage must be permeable to water vapor, but not permeable to liquid water, microorganisms and particles of dirt. The material should be anisotropic. By anisotropic is meant that the fabric stretches more in one direction than in the other. This characteristic allows the dressing to stretch in the direction of the skin to which it is applied and also allows for easy application.

The material should also be thin so that the dressing is not easily bumped or displaced by contact with outside sources. Other bandages which use a knitted fabric result in a very voluminous bandage. See, for example, U.S. Patent No. 4,236,550 to Braun et al. and U.S. Patent No. 4,391,106 to Schafer et al. Thinner wound dressings presently available of polyurethane often require two or more trained medical personnel for proper application because of its thinness, elasticity and tendency to stick to itself during application.

## SUMMARY OF THE INVENTION

The present invention is a wound dressing formed of an ultrathin polyurethane membrane which allows light and oxygen to reach the wound while serving as a barrier to bacteria. The wound dressing includes a textile reinforcement fabric within a layer of crosslinked aliphatic polyurethane material. The fabric enables the wound dressing to be made very thin and yet be strong and anisotropic.

It is therefore an object of the present invention to provide a wound dressing which is strong, yet flexible, and which can be made to conform to the shape of the site of the wound.

It is still a further object of the present invention to provide such a material for use as a wound dressing which is anisotropic and thin.

It is still a further object of the present invention to provide a material which can be easily formed and has sufficient support that it retains its shape so that it can easily be applied to a wound by one person in adverse circumstances.

The foregoing and other objects and features of

4

the claimed invention will be understood by those skilled in the art from a reading of the description which follows.

### BRIEF DESCRIPTION OF THE DRAWING

FIG. 1 is a view of the anisotropic wound dressing of the present invention which had been made into a pressure sensitive, self-adhesive first aid dressing.

FIG. 2 is a cross-sectional view of the dressing in Fig. 1, taken along the cut at line 2-2, exposing the layers of the dressing.

FIG. 3 is a view of the weave of the reinforcing fabric structure of the anisotropic wound dressing of the present invention, magnified 50 times.

### DESCRIPTION OF THE PREFERRED EMBODIMENTS

A wound dressing in preferred embodiment consists of an open mesh knitted fabric which is embedded in a crosslinked aliphatic polyurethane. The polyurethane-forming solution is applied to the fabric as a film, occluding the interstices of the fabric. The polyurethane is then cured by exposure to ultraviolet light. One side of the fabric coated with cured polyurethane may optionally be provided with a pressure sensitive adhesive.

The open mesh knitted fabric is used as a reinforcement. The term "knitted" is intended to describe the process to form textile material by interlacing yarn or threads in a series of connected loops with needles. The reinforcing fabric can be made out of any textile material, such as a polyester. Polyester, however, is considered to be very expensive. A very porous fabric, which is less expensive than a polyester, is the preferred knit fabric. A desirable fabric is a warp knit 15 denier nylon tricot, heat set material. One such fabric is sold by Gehrring Textiles, New York, New York, and is formed from Nylon 6-6 yarns. Any fabric of the proper geometry, however, which is biocompatible may be used.

The preferred knitted fabric has the following characteristics. The openings in the fabric are hexagonal, of a size ranging from 0.5 mm to 4 mm across, although preferably they are 2 mm in size. The fabric is knitted from yarns having a diameter in the range of 0.025-0.203 mm, although preferably with a diameter of 0.1 mm. The term "yarn" as used herein is intended to describe both yarns formed from fibers or filaments twisted together and yarns formed from monofilament fibers. The yarn itself need only have modest elongation properties. The stretch characteristics of the fabric is achieved by the mechanical processing and geometry.

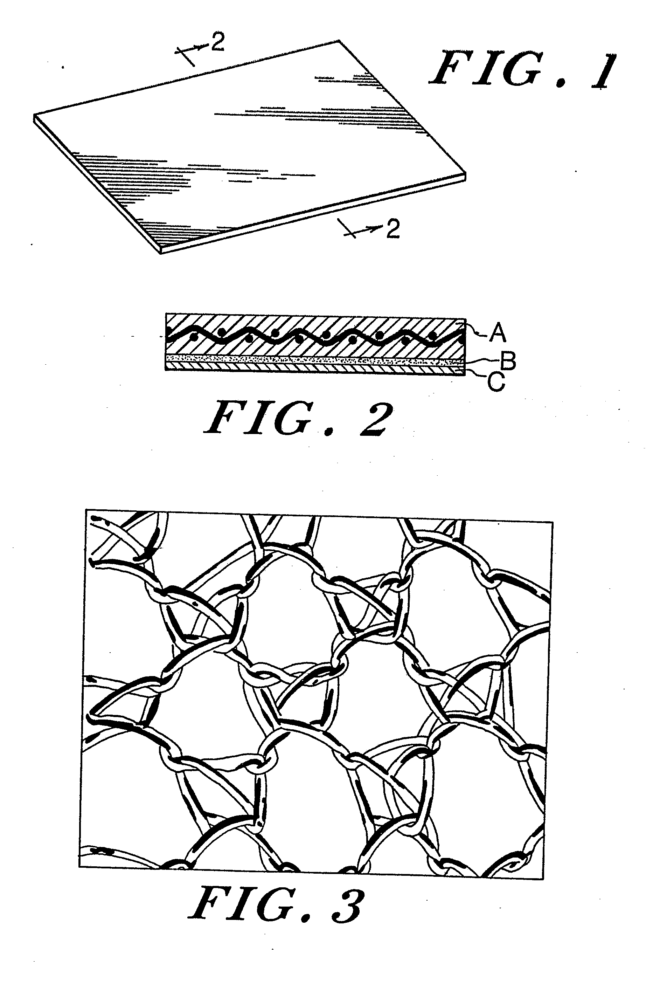

The fabric described above is shown in Fig. 3, magnified 50 times. The small thread to large open space ratio is considered important because it minimizes the obstruction of light and moisture, it provides high tear characteristics, suppleness and contributes to the anisotropic tendencies. In its preferred embodiment, the ratio of the diameter of the thread to the size of the void area is 0.1:2, or 5% thread to 95% void area.

An aliphatic polyurethane solution is then applied to the fabric. Any non-thermoplastic aliphatic polyurethane that is bio-compatible is suitable, although one which is also drug dispensing is especially preferred. The preferred formation is the reaction product of isophorone diisocyanate and a macroglycol which results in an isocyanate terminated prepolymer. The prepolymer is then reacted with a chain terminator to form a vinyl terminated polyurethane oligomer. Optionally, a photosensitizer may be admixed with the foregoing compounds at any point prior to curing to form a homogeneous admixture, which is applied to the fabric.

The isophorone diisocyanate (IPDI) used in the present invention is an aliphatic compounds having the following formula:

IPDI is utilized in the present invention because it is a liquid at room temperature and thus, easily coats the fabric, and because it cures to a crystal clear product upon exposure to ultraviolet light, rather than yellowing as is the case with many prior art diisocyanates, and because it cures without any temperature rise.

The macroglycol preferred for use in the present invention is a polypropylene glycol (PPG), preferably having a molecular weight of 500-5000 Daltons and, more preferably 1000-3000 Daltons. PPG is preferred because it reacts with the IPDI at a fast rate at room temperature with no temperature rise. Other high molecular weight glycols such as polyethylene glycol (PEG) may be employed, but PEG is a solid at room temperature and a feasible rate of reaction would require heating. As used herein, the term "macroglycol" has reference to any glycol having a molecular weight in excess of 500 Daltons.

The chain terminator used in formulating the products of the invention should have both hydroxyl and vinyl functional groups and is preferably an acrylic compound such as hydroxyethyl acrylate or hydroxyethyl methacrylate. Hydroxyethyl methacrylate (HEMA) is most preferred for use as the chain terminator.

The present invention may be used with or without a drug dispensing polyurethane. Should the drug dispensing characteristics be desired in the final product, a large variety of drugs, including heat labile drugs, may be incorporated into the compositions by the present invention at any point in the formulation/reaction sequence because the process referenced above does not involve an exothermic reaction.

Photosensitizers useful herein include benzophenone, acetophenone, azobenzene, acenaphthenequinone, o-methoxy benzophenone, thioxanthen-9-one, xanthen-9-one, 7-H-Benz(de) anthracen-7-one, 1-naphthaldehyde 4,4'-bis (dimethylamino)benzophenone, fluorene-9-one, 1'-acetonaphthone, 2'-acetonaphthone, anthraquinone, 2-tern.-butyl anthraquinone, 4-morpholino-benzophenone, p-diacetylbenzene, 4-aminobenzophenone, 4'-methoxyacetophenone, diethoxyacetophenone, benzaldehyde, and the like.

Specifically useful herein are acetophenone photosensitizers of the structure:

wherein R is alkyl of from 1 to about 8 carbon atoms, or aryl of 6 ring carbon atoms and R' is hydrogen, alkyl of from 2 to about 8 carbon atoms, aryl of from 6-14 carbon atoms, or cyclo alkyl of 5 to 8 ring carbon atoms.

Diethoxyacetophenone is the preferred photosensitizer.

The diisocyanate, macroglycol and chain terminator are reacted in approximately stoichiometric amounts, i.e., in the approximate ratio of 2 moles (2.0 equiv.) isophorone diisocyanate to 1 mole (1.0 equiv.) macroglycol to 2 moles (1.0 equiv.) chain terminator. At the end of the reaction between the prepolymer and the chain terminator free isocyanate is monitored by infrared spectrophotometry and, if necessary, additional small amounts of the chain terminator may be added to scavenge any remaining isocyanate. It is important that the low molecular weight monomers present in the composition be reacted prior to contact with the skin so that only compounds with molecular weights of 1500-5000 Daltons are present. The high molecular weight compounds do not leach out of the wound dressing into the underlying tissue and are therefore non-toxic.

An antioxidant such as tetrakis (methylene (3,5-di-tert-butyl-4-hydroxyhydrocinnamate)) may be added to inhibit spontaneous oxygen-initiated curing.

A polyurethane catalyst such as dioctyl tin dilaurate, N-methyl morpholine, trimethylamine, triethylamine, zinc octoate, or dibutyl tin dilaurate is added to both the reaction medium in which the prepolymer is formed and the reaction medium in which the prepolymer is reacted with the chain terminator.

The above polyurethane solution is applied to the fabric described above. This coating step is preferably done by dipping the fabric in the polyurethane solution. The polyurethane coats the fabric fibers and fills the interstices. The thickness of the polyurethane membrane or film sandwiching the fabric is 0.0254 mm or less and preferably is 0.01 mm.

Curing may be accomplished by ultraviolet radiation, typically between 219 and 425 nm for 20 seconds at 0.5 W/cm$^2$. Curing transforms the liquid oligomer into a solid elastomer.

It should be understood that in addition to curing an oligomer, it is also possible to utilize a cured polymer in solution followed by evaporating the solvent while the polyurethane is held by the reinforcing fabric. The two foregoing systems are referred to as "polyurethane-forming solutions."

The fabric coated with cured polyurethane is clear, soft and elastic. The resulting product is also thin, oxygen permeable, anisotropic, strong, does not wrinkle, and therefore, keeps its shape. Because there is no gauze, bacteria buildup is prevented.

Any pressure sensitive adhesive conventionally used for wound dressings or bandages may be spread over one surface of the fabric coated with cured polyurethane, e.g. a polyacrylate adhesive or a polyvinylethyl ether blend adhesive. A release paper or plastic film is then applied over the exposed surface of the adhesive.

Figure 2 is the cross-sectional view of a first aid dressing made from the anisotropic wound dressing of the present invention. The cross-sectional view exposes the various layers of the dressing: A is the coated knitted reinforcing fabric, B is the pressure sensitive adhesive and C is the release paper or plastic film.

The invention may be embodied in other specific forms without departing from the spirit or essential characteristics thereof. The present embodiments are therefore to be considered in all respects as illustrative and not restrictive, the scope of the invention being indicated by the appended claims rather than by the foregoing despcription, and all changes which come within the meaning and range of equivalency of the claims are therefore intended to be embraced therein.

What is claimed is:

1. An anisotropic wound dressing comprising a knitted reinforcing fabric defining a network of interstices having a void area between the range of 0.5 mm to 4 mm across and formed from yarns having a diameter in the range of 0.025 to 0.203 mm, said reinforcing fabric having a layer or film of crosslinked non-thermoplastic polyurethane, the thickness of said layer being 0.254 mm or less.

2. The wound dressing of claim 1 where said void area of said knitted reinforcing fabric is 2 mm across.

3. The wound dressing of claim 1 where said layer of crosslinked non-thermoplastic polyurethane is 0.01 mm.

4. The wound dressing of claim 1 where said knitted reinforcing fabric is formed from yarns having a diameter of 0.1 mm.

5. The wound dressing of claim 1 where said knitted reinforcing fabric is a material formed from Nylon 6-6 yarns.

6. The wound dressing of claim 1 further comprising a coating of pressure sensitive adhesive on one surface of said coated fabric.

7. The wound dressing of claim 1 where said polyurethane is the reaction product of

        1) isophorone diisocyanate;

        2) a macroglycol; and

        3) a monomer containing hydroxyl and vinyl groups.

8. The wound dressing of claim 1 wherein said polyurethane coated fabric has been cured by incorporation of a photosensitizer and exposure to ultraviolet light.

9. The wound dressing of claim 7 wherein said dressing is formed by

1) reacting a isophorone diisocyanate and said macroglycol together in the presence of a catalyst to form an isocyanate terminated prepolymer;

2) reacting said prepolymer with said monomer containing hydroxyl and vinyl groups to form an ultraviolet-curable, vinyl terminated polyurethane oligomer;

3) admixing said oligomer with the pharmacoactive agent to form an UV-curable homogeneous blend;

4) dipping a knitted reinforcing anisotropic fabric in said blend; and

5) curing said film by exposure to ultraviolet light.

10. The wound dressing of claim 9 further comprising a coating of a pressure sensitive adhesive on one surface of said coated fabric.

0199857

FIG. 1

FIG. 2

A
B
C

FIG. 3

## EUROPEAN SEARCH REPORT

EP 85201735.9

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl.4) |
| A | GB - A - 1 476 894 (AMERICAN CYANAMID)<br>* Claims 1,3 *<br>-- | 1 | A 61 L 15/01<br>A 61 L 15/06<br>A 61 L 15/03<br>A 61 F 13/00<br>B 32 B 27/12<br>C 08 G 18/67 |
| A | US - A - 3 949 742 (NOWAKOWSKI)<br>* Claims 1,4 *<br>-- | 1,3 | |
| A | US - A - 4 051 848 (LEVINE)<br>* Claims 1,3 *<br>-- | 1,5 | |
| A | GB - A - 2 093 702 (SMITH AND NEPHEW)<br>* Claims 1,4,7 *<br>-- | 1,6 | |
| D,A | US - A - 3 870 593 (ELTOW et al.)<br>* Claims 1,2 *<br>-- | 1,6 | |
| A | US - A - 4 264 757 (PARK)<br>* Claim 1; column 3, lines 6,7; column 5, line 66 - column 6, line 4 *<br>-- | 7-9 | TECHNICAL FIELDS SEARCHED (Int. Cl.4)<br><br>A 61 L<br>A 61 F<br>C 08 G 18/00<br>D 06 N<br>B 32 B |
| A | EP - A1 - 0 114 581 (SOGIMI)<br>---- | | |

The present search report has been drawn up for all claims

| Place of search | Date of completion of the search | Examiner |
|---|---|---|
| VIENNA | 04-08-1986 | FARNIOK |